## Europäisches Patentamt

## European Patent Office

## Office européen des brevets

(11) Numéro de publication: **0 178 197**
**B1**

(19)

# FASCICULE DE BREVET EUROPÉEN

(12)

(45) Date de publication du fascicule du brevet:
**31.05.89**

(51) Int. Cl.⁴: **A 61 B 5/08**, A 61 B 5/10

(21) Numéro de dépôt: **85401679.7**

(22) Date de dépôt: **23.08.85**

(54) **Dispositif pour la surveillance du rythme respiratoire, notamment pour la détection d'apnée chez les nourrissons.**

(30) Priorité: **11.09.84 FR 8413915**

(43) Date de publication de la demande:
**16.04.86 Bulletin 86/16**

(45) Mention de la délivrance du brevet:
**31.05.89 Bulletin 89/22**

(84) Etats contractants désignés:
**AT BE CH DE GB IT LI LU NL SE**

(56) Documents cité:
**EP-A-0 019 321**
**WO-A-80/00054**
**FR-A-2 377 793**
**FR-A-2 513 507**
**US-A-3 730 173**
**US-A-4 245 651**

(73) Titulaire: **Fournier Montgieux, François, 15, rue Palapharnerie, F-84000 Avignon (FR)**

(72) Inventeur: **Fournier Montgieux, François, 15, rue Palapharnerie, F-84000 Avignon (FR)**

(74) Mandataire: **Marquer, Francis, 35, Avenue Victor Hugo Résidence Chamfleury, F-78180 Voisins- le- Bretonneux (FR)**

EP 0 178 197 B1

LIBER, STOCKHOLM 1989

## Description

La présente invention concerne un dispositif pour la détection en continu du rythme respiratoire, notamment mais non exclusivement, en vue de la prévention de la mort subite du nourrisson par arrêt respiratoire ou apnée au cours du sommeil.

On sait en effet que l'apnée du nourrisson constitue, à l'heure actuelle, la cause principale de décès d'enfants de moins d'un an. Ce syndrome reste encore inexpliqué et il n'existe pas de moyen de prévention véritablement efficace.

La seule action possible qui concerne surtout la prévention des récidives, consiste en une surveillance cardiorespiratoire continue du nourrisson pendant son sommeil, de manière à pouvoir détecter une absence de respiration et, si elle se prolonge, procéder à des manoeuvres de secourisme élémentaires telles que secousses, respiration artificielle ou même, dans certains cas, massage cardiaque externe. Du fait que ce syndrome peut intervenir après seulement quelques minutes de sommeil, il s'avère nécessaire, si l'on veut exclure toute possibilité d'accident, d'exercer cette surveillance du nourrisson pratiquement vingt quatre heures sur vingt quatre.

En conséquence, dans l'état actuel de la technique, une telle surveillance pour être entièrement efficace, ne peut être réellement assuree qu'en milieu hospitalier, à l'aide d'appareillages relativement coûteux nécessitant la présence constante d'un auxiliaire médical. Or, la durée de prise en charge du nourrisson en milieu hospitalier est souvent insuffisante pour éliminer tout risque et ce, d'autant plus que la décision d'arrêt de la surveillance permanente est habituellement difficile à prendre par manque de critères formels de disparition du risque.

Dans le but de pallier à ces inconvénients, il a déjà été proposé des dispositifs permettant d'exercer à domicile une surveillance respiratoire du nourrisson. Ces dispositifs font habituellement intervenir un boîtier électronique ou "moniteur" relié par une liaison, par exemple pneumatique ou électrique, à un ou plusieurs détecteurs destinés à être disposés sur le corps du nourrisson ou à proximité immédiate de celui-ci. Un tel dispositif a été proposé par US-A-4 245 651.

Toutefois, un premier inconvénient de ces dispositifs, outre le fait qu'ils demeurent coûteux, consiste en ce que les liaisons entre le moniteur et l'enfant, rendent leur utilisation peu commode, notamment lors de l'habillage, du déshabillage et des déplacements du nourrisson. En outre, le nourrisson en bougeant peut exercer des tractions sur les connexions et risque de déplacer ou de détériorer les capteurs en les rendant inefficaces, ce qui peut engendrer de fausses alarmes.

Un deuxième inconvénient consiste en ce que les capteurs et, éventuellement, leurs liaisons au boîtier électronique, doivent être régulièrement changés, ce qui implique une augmentation non négligeable du coût de l'utilisation du dispositif.

Un troisième inconvénient provient du fait qu'en vue de permettre la surveillance, ces appareils produisent habituellement des impulsions lumineuses et/ou sonores (bips) en synchronisme avec le rythme respiratoire de l'enfant. En conséquence, même dans le cas où ils comprennent des moyens permettant d'engendrer un signal d'alarme (en général sonore) pour une absence de respiration dépassant une durée prédéterminée, ils nécessitent la présence permanente d'une personne à proximité immédiate de l'appareil.

L'invention a donc pour but de supprimer tous ces inconvénients.

Elle propose à cet effet un appareil du type comprenant un dispositif de détection et de signalisation du rythme respiratoire de l'enfant muni de moyens permettant de déceler une pause respiratoire anormale et d'engendrer un signal d'alarme lorsque cette pause depasse une durée prédéterminée, cet appareil étant plus particulièrement caractérisé en ce que:

- le dispositif de détection et de signalisation est autonome et est logé dans un boîtier de petites dimensions comprenant une paroi d'application dont la face extérieure est destinée à être maintenue appliquée sur la peau de l'enfant, en un emplacement convenablement choisi, ladite paroi comportant au moins une zone de détection mobile sous l'effet des déplacements de la peau engendrés par le processus respiratoire de l'enfant;
- ce dispositif de détection et de signalisation comprend un transducteur sensible aux mouvements de la zone de détection;
- le boîtier est équipé de moyens de fixation sur le corps de l'enfant, aptes à maintenir ladite face extérieure de la paroi d'application et, en particulier, de la zone de détection, contre la peau de l'enfant.

L'invention ne se limite pas à un type de transducteur particulier, étant entendu que celui-ci doit permettre de transformer en un signal électrique utilisable par le dispositif autonome de détection et/ou de signalisation, les déplacements et/ou les variations de pression (mouvements thoraciques et/ou abdominaux), produits au niveau de la peau, à l'emplacement considéré (par exemple à proximité de l'ombilic), au cours du cycle respiratoire.

Il convient de noter que les organes de détection agissent en combinaison avec les moyens de fixation du boîtier sur le corps de l'enfant. En effet, outre leur fonction de fixation, ces moyens de fixation contribuent à fournir au transducteur, par l'intermédiaire du boîtier, une position et/ou une pression de référence indispensable pour obtenir une réponse significative. En outre, l'inertie de l'ensemble du boîtier peut egalement contribuer à l'acquisition de ces paramètres de référence.

Avantageusement, ces moyens de fixation pourront consister en un adhésif prévu à la périphérie de la face d'application du boîtier et destiné à agir directement sur la peau de l'enfant ou en une ceinture, élastique ou inextensible, destinée à ceindre le corps de l'enfant.

Par ailleurs, le dispositif de détection et de signalisation peut comprendre des moyens permettant de produire, à l'image des dispositifs connus, des impulsions lumineuses et/ou sonores à chacun des cycles respiratoires, ainsi qu'une alarme sonore en cas d'arrêt respiratoire prolongé grâce à des moyens de signalisation prévus dans le boîtier.

Toutefois, pour éviter les inconvénients précédemment mentionnés de ces moyens de signalisation qui sont accrus du fait que le boîtier se trouve la plupart du temps recouvert par les vêtements de l'enfant, l'invention prévoit en outre, utilisés séparément ou en combinaison:

- un dispositif de réveil ou de réanimation de l'enfant pouvant par exemple faire intervenir des électrodes au travers desquelles sont produites des décharges de courant, et/ou
- un émetteur (par exemple haute fréquence) placé dans le boîtier et associé à au moins un récepteur portatif affecté à la personne qui assure la surveillance de l'enfant.

Ces deux dispositifs sont commandés en réponse à l'émission du susdit signal d'alarme, éventuellement après un intervalle de temps présélectionné.

En outre, le dispositif de détection et de signalisation peut comprendre des moyens pour:

- la présélection de l'intervalle de temps choisi entre l'arrêt de la respiration et l'émission du signal d'alarme (par exemple de cinq secondes à quarante secondes par pas de cinq secondes); et/ou
- la présélection de l'intervalle de temps choisi entre l'arrêt de la respiration et le déclenchement du dispositif de réveil de l'enfant (de vingt à soixante secondes par pas de cinq secondes); et/ou
- l'inhibition du signal sonore émis à chaque cycle de respiration de l'enfant; et/ou
- l'acquittement automatique (arrêt/réarmement) de l'alarme sonore et/ou du dispositif de réveil de l'enfant à la suite d'une nouvelle respiration; et/ou
- l'inhibition du dispositif de réveil de l'enfant; et/ou
- le comptage et la visualisation sur demande, du nombre de pauses respiratoires supérieures à un intervalle de temps présélectionné avec possibilité de remise à zéro du compteur; et/ou
- le contrôle de l'état de la pile utilisée avec alarme sonore en cas d'usure de celle-ci; et/ou
- le contrôle du bon fonctionnement de l'électronique par un circuit électronique indépendant, avec alarme sonore en cas d'anomalie de fonctionnement.

Un mode de réalisation de l'invention sera décrit ci-après, à titre d'exemple non limitatif, avec référence aux dessins annexés dans lesquels:

La figure 1 est une coupe axiale schématique d'un dispositif selon l'invention.

La figure 2 est une représentation schématique d'un capteur de déplacement à électret utilisé dans le dispositif représenté figure 1.

Les figures 3 et 4 sont des vues schématiques illustrant le principe du détecteur de déplacement utilisé dans le dispositif de la figure 1.

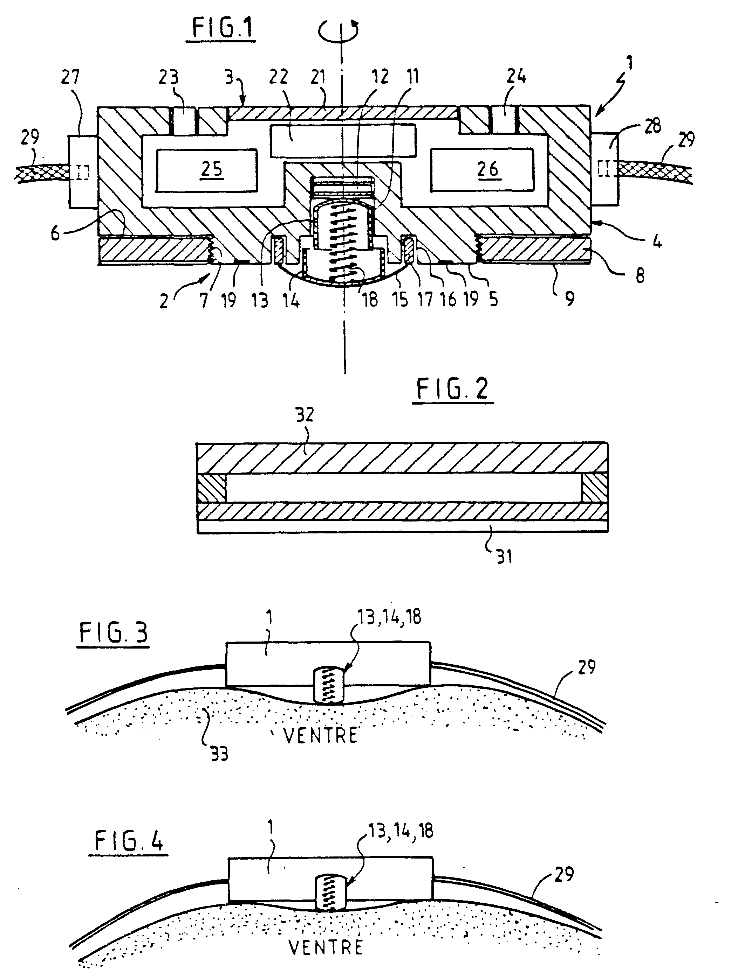

Tel que représenté sur la figure 1, le dispositif pour la détection en continu du rythme respiratoire d'un enfant comprend tout d'abord un boîtier cylindrique 1 d'une taille légèrement supérieure à celle d'un boîtier de montre (diamètre de l'ordre de quelques cm). D'une façon plus générale, la forme de ce boîtier 1 et le choix du matériau utilisé sont prévus de manière à ce que ce boîtier 1 puisse rester sur l'enfant en permanence sans aucune gêne pour ce dernier (irritation, douleur, etc...)

Ce boîtier 1 comporte plus particulièrement une face d'application 2, une face de visualisation 3 opposée à la face d'application 2 et une paroi latérale cylindrique 4.

La face d'application 2 du boîtier 1 comporte une zone centrale circulaire 5, reliée à une zone périphérique en forme de couronne 6 située en retrait, par un épaulement 7 muni d'un filetage. Sur ce filetage peut venir se visser une couronne 8 dont la face inférieure qui vient dans le plan de la zone centrale, en fin de vissage, porte une garniture adhésive 9.

Au centre de la zone 5 est prévu un évidement cylindrique coaxial 11 à l'intérieur duquel est monté un détecteur comprenant:

- un capteur de déplacement 12 logé au fond de l'évidement,
- un premier poussoir 13 monté coulissant dans le logement 11 et venant porter sur le capteur de déplacement 12,
- un deuxième poussoir 14 monté coulissant dans le logement 11 et retenu par une membrane souple 15 montée de façon étanche sur la zone centrale 5 de la face de fixation 2, grâce à une gorge circulaire 16 dans laquelle vient se fixer la bordure périphérique 17 de la membrane, et
- un ressort de pression 18 disposé entre les deux poussoirs 13, 14.

Sur la zone centrale 5 sont en outre prévues une ou plusieurs électrodes 19 par lesquelles il est possible de transmettre à l'enfant une décharge de courant en vue de le réanimer (réveiller).

La face supérieure 3 du boîtier 1 comprend quant à elle une fenêtre transparente 21 au droit de laquelle sont disposés un afficheur 22, une source lumineuse 23 et une source sonore 24.

Bien entendu, le boîtier peut également comprendre des moyens tels que des poussoirs d'interruption ou des touches sensitives permettant la mise en marche de l'appareil et les différentes présélections et commandes nécessaires au paramétrage et au fonctionnement de l'appareil. Il comprend en outre des moyens d'accès à une ou plusieurs piles 25 servant à l'alimentation du circuit électronique 26 contenu dans le boîtier.

Ce circuit électronique qui est couplé au transducteur 12, à l'afficheur 22, aux sources lumineuse et sonore 23 et 24, aux électrodes 19 ainsi qu'aux poussoirs des interrupteurs de commande (non représentés) comprend tous les éléments nécessaires au fonctionnement de l'appareil. Ce circuit qui fait intervenir des moyens connus de l'électronicien, et éventuellement un microprocesseur ainsi qu'un émetteur haute fréquence, ne constitue pas l'objet de l'invention et ne sera donc pas décrit.

Sur la paroi latérale 4 du boîtier 1 sont prévus, en deux emplacements diamétralement opposés, des moyens de fixation 27, 28 d'une ceinture abdominale 29 éventuellement élastique.

Dans cet exemple, la détection de la respiration est basée sur l'utilisation, en tant que transducteur 12, d'un micro à électret constitué, comme représenté sur la figure 2, par un électret métallisé 31 placé en regard d'une électrode 32 à une distance de quelques dizaines de microns.

L'électret 31 étant chargé, tout déplacement de celui-ci par rapport à l'électrode 32 induit un courant (entre l'électret et l'électrode) provoqué par une augmentation des charges positives ou négatives sur l'électrode 32. Les charges induites sur l'électrode 32 sont donc proportionnelles à l'amplitude du déplacement de l'électret 31.

Dans cet exemple, l'invention utilise ce principe, étant entendu que, dans ce cas, le déplacement de l'électret 31 n'est pas provoqué par une vibration sonore comme dans le cas d'un micro, mais par l'ensemble constitué par les poussoirs 13 et 14 et le ressort 18.

Comme illustré sur les figures 3 et 4, avant la respiration, la zone du ventre 33 de l'enfant sur laquelle est placé le boîtier 1, est détendue et n'exerce sur l'ensemble poussoirs 13, 14/ressort 18 qu'une faible pression (figure 3).

Au moment de la respiration, le gonflement de l'abdomen de l'enfant qui est en partie absorbé par l'élasticité de la ceinture abdominale 29, entraîne un déplacement axial du poussoir 14.

Ce déplacement est transmis de façon élastique au poussoir 13 par l'intermédiaire du ressort de compression 18 et, par conséquent, à l'électret 31 qui délivre alors au circuit électronique 26, un signal proportionnel à l'amplitude de ce déplacement.

Il convient de noter que l'utilisation de l'ensemble poussoirs 13, 14/ressort 18 pour transmettre l'effort exercé par l'abdomen jusqu'à l'électret 31, est destinée à éviter tout risque de détérioration de l'électret 31.

Bien entendu, l'invention ne se limite pas à ce mode de transmission. En effet, la sollicitation de l'électret 31 pourrait par exemple s'effectuer par une transmission hydraulique faisant intervenir un fluide hydraulique logé dans la cavité fermée délimitée par la membrane 15 et l'évidement 11, ainsi que par un piston mu par le fluide hydraulique et venant porter sur le micro à électret.

De même, le micro à électret pourrait consister en tout autre capteur de pression et/ou de déplacement. Il pourrait en outre consister en un organe de détection à ultrasons, le fluide hydraulique étant alors remplacé par un fluide assurant la transmission directe avec, éventuellement, une focalisation des ultrasons sur l'organe de détection.

Un avantage important des appareils selon l'invention réside dans le fait qu'il peut fonctionner sans l'adjonction de matériel consommable (électrode, gel de contact, etc...) à l'exception de la pile 25 qui devra être renouvelée régulièrement.

**Revendications**

1. Appareil pour la détection, en continu, du rythme respiratoire d'un enfant, notamment en vue de la prévention de la mort subite du nourrisson, par arrêt respiratoire au cours du sommeil, cet appareil comprenant un dispositif de détection et de signalisation du rythme respiratoire de l'enfant, muni de moyens permettant de déceler une pause respiratoire anormale et d'engendrer un signal d'alarme lorsque cette pause dépasse une durée prédéterminée, caractérisé en ce que:
   - le dispositif de détection et de signalisation est autonome et est logé dans un boîtier (1) de petites dimensions comprenant une paroi d'application dont la face extérieure est destinée à être maintenue appliquée sur la peau de l'enfant, en un emplacement convenablement choisi, ladite paroi comportant au moins une zone de détection (15) mobile sous l'effet des déplacements de la peau engendrés par le processus respiratoire de l'enfant;
   - ce dispositif de détection et de signalisation comprend un transducteur (12, 13, 18) sensible aux mouvements de la zone de détection;
   - le boîtier (1) est équipé de moyens de fixation (8, 9, 28, 29) sur le corps de l'enfant, aptes à maintenir ladite face extérieure (2) de la paroi d'application et, en particulier, de la zone de détection (15), contre la peau de l'enfant.

2. Appareil selon la revendication 1, caractérisé en ce que le susdit transducteur (12) comprend un micro à électret sur lequel sont retransmis, par des moyens de transmission, les déplacements et/ou les variations de pression exercées au niveau de la peau de l'enfant en contact avec la surface d'application du boîtier (1).

3. Appareil selon la revendication 2,
caractérisé en ce que lesdits moyens de transmission sont mécaniques ou hydrauliques.

4. Appareil selon l'une des revendications 2 et 3,
caractérisé en ce que ledit transducteur et lesdits moyens de transmission sont logés dans un évidement (11) formé dans la paroi d'application et.refermé au niveau de la face extérieure (2) par une membrane souple (5).

5. Appareil selon la revendication 4,
caractérisé en ce que lesdits moyens de transmission comprennent une premier poussoir mobile (13) venant porter contre le susdit transducteur (12), et un deuxième poussoir (14) maintenu appliqué contre ladite membrane (15), et en ce qu'entre les deux poussoirs (13, 14) est disposé un ressort (18).

6. Appareil selon l'une des revendications précédentes,
caractérisé en ce que lesdits moyens de fixation du boîtier (1) consistent en un adhésif (9) prévu à la périphérie de la face d'application (11) du boîtier (1) et destiné à agir directement sur la peau de l'enfant.

7. Appareil selon la revendication 6,
caractérisé en ce que ledit adhésif (9) est monté sur une pièce (8) amovible venant se fixer sur la paroi d'application (11) du boîtier (1).

8. Appareil selon la revendication 7,
caractérisé en ce que ladite pièce consiste en une couronne (8) venant se visser sur un filetage ménagé sur un épaulement (7) de la face extérieure (11) de la paroi d'application du boîtier (1).

9. Appareil selon l'une des revendications précédentes,
caractérisé en ce que le boîtier (1) comprend des moyens de fixation (28) d'une ceinture abdominale (29).

10. Appareil selon l'une des revendications précédentes,
caractérisé en ce que le susdit dispositif de détection et de signalisation comprend des moyens de signalisation (22, 23, 24) prévus dans le boîtier permettant de produire des impulsions lumineuses et/ou sonores à chacun des cycles de respiration.

11. Appareil selon l'une des revendications precedentes,
caractérisé en ce qu'il comprend en outre un dispositif de réveil ou de réanimation (19) de l'enfant commandé par un signal émis par le dispositif autonome de détection et de signalisation lorsqu'une pause respiratoire dépasse une durée prédéterminée.

12. Appareil selon la revendication 11,
caractérisé en ce que ledit dispositif de réveil ou de réanimation comprend au moins une électrode (19) placée sur la face extérieure (11) de la paroi d'application du boîtier (1) et par laquelle peuvent être produites des décharges électriques commandées par ledit signal.

13. Appareil selon l'une des revendications précédentes,
caractérisé en ce qu'il comprend un émetteur haute fréquence placé dans le boîtier et destiné à transmettre à distance le signal produit à partir desdits organes de détection et/ou le susdit signal d'alarme à au moins un récepteur affecté à la personne qui assure la surveillance de l'enfant.

14. Appareil selon l'une des revendications précédentes,
caractérisé en ce qu'il comprend en outre des moyens pour
- la présélection de l'intervalle de temps choisi entre l'arrêt de la respiration et l'émission du signal d'alarme; et/ou
- la présélection de l'intervalle de temps choisi entre l'arrêt de la respiration et le déclenchement du dispositif de réveil de l'enfant; et/ou
- l'inhibition du signal sonore émis à chaque cycle de respiration de l'enfant; et/ou
- l'acquittement automatique (arrêt/réarmement) de l'alarme sonore et/ou du dispositif de réveil de l'enfant à la suite d'une nouvelle respiration; et/ou
- l'inhibition du dispositif de réveil de l'enfant; et/ou
- le comptage et la visualisation sur demande, du nombre de pauses respiratoires supérieures à un intervalle de temps présélectionné avec possibilité de remise à zéro du compteur; et/ou
- le contrôle de l'état de la pile utilisée avec alarme sonore en cas d'usure de celle-ci; et/ou
- le contrôle du bon fonctionnement de l'électronique par un circuit électronique indépendant, avec alarme sonore en cas d'anomalie de fonctionnement.

**Patentansprüche**

1. Gerät zur kontinuierlichen Überwachung der Atemfrequenz eines Kindes, insbesondere zur Verhütung des plötzlichen Todes eines Säuglings wegen Atemstillstandes während des Schlafes, wobei besagtes Gerät eine Erfassungsund Anzeigevorrichtung der Atemfrequenz des Kindes aufweist, die mit Mitteln versehen ist, um eine anormale Atempause zu erfassen und ein Alarmsignal auszulösen, wenn diese Pause eine vorbestimmte Dauer überschreitet, dadurch gekennzeichnet, dass
- die Erfassungs- und Anzeigevorrichtung autonom und in einem Gehäuse (1) mit kleinen Ausmassen untergebracht ist, welches eine Auflagewandung aufweist, deren Aussenseite gegen die Haut des Kindes an einer vorteilhaft gewählten Stelle angelegt wird, wobei besagte Wandung mindestens eine Erfassungszone (15) aufweist, die infolge der durch die Atembewegungen des Kindes verschobenen Haut beweglich ist;
- und die Erfassungs- und Anzeigevorrichtung einen auf die Bewegungen der Erfassungszone ansprechenden Wandler (12, 13, 18) aufweist;
- das Gehäuse (1) mit Mitteln (8, 9, 28, 29) zur Befestigung auf dem Körper des Kindes versehen ist, um besagte Aussenseite (2) der Auflagewandung und insbesondere die Erfassungszone (15) gegen die Haut des Kindes anzulegen.

2. Gerät nach Anspruch 1, dadurch gekennzeichnet, dass besagter Wandler (12) ein Mikro mit einem Elektreten aufweist, an das, durch Übertragungsmittel, die Verschiebungen und/oder Druckunterschiede auf die Haut des Kindes die mit der Erfassungszone des Gehäuses (1) in Kontakt steht, übermittelt werden.

3. Gerät nach Anspruch 2, dadurch gekennzeichnet, dass besagte Überwachungsmittel mechanisch oder hydraulisch sind.

4. Gerät nach einem der Ansprüche 2 oder 3, dadurch gekennzeichnet, dass besagter Wandler und besagte Übertragungsmittel in einer Aussparung (11) untergebracht sind, die in der Auflagewandung angeordnet ist und auf der Aussenseite (2) durch eine elastische Membran (5) verschlossen ist.

5. Gerät nach Anspruch 4, dadurch gekennzeichnet, dass besagte Übertragungsmittel einen ersten beweglichen Drücker (13) aufweisen, der gegen besagtenWandler (12) zur Anlage kommt und einen zweiten Drücker (14), der ständig gegen besagte Membran (15) anliegt, und dass zwischen beiden Drückern (13, 14) eine Feder (18) angeordnet ist.

6. Gerät nach einem der vorhergehenden Ansprüche, dadurch gekennzeichnet, dass besagte Mittel zur Befestigung des Gehäuses (1) aus einem Haftmittel (9) bestehen, das am Umfang der Auflagewandung (11) des Gehäuses (1) vorgesehen ist und direkt auf die Haut des Kindes wirkt.

7. Gerät nach Anspruch 6, dadurch gekennzeichnet, dass besagtes Haftmittel (9) auf einem abnehmbaren Teil (8) angebracht ist, das auf der Auflagewandung (11) des Gehäuses (1) befestigt ist.

8. Gerät nach Anspruch 7, dadurch gekennzeichnet, dass besagtes Teil aus einem Ring (8) besteht, der auf ein auf einem Absatz (7) der Aussenseite (11) der Auflagewandung des Gehäuses (1) angebrachtes Gewinde aufgeschraubt werden kann.

9. Gerät nach einem der vorhergehenden Ansprüche, dadurch gekennzeichnet, dass das Gehäuse (1) Mittel (28) zur Befestigung eines Bauchgürtels (29) aufweist.

10. Gerät nach einem der vorhergehenden Ansprüche, dadurch gekennzeichnet, dass besagte Erfassungs - und Anzeigevorrichtung im Gehäuse vergesehene Signalisiermittel (22, 23, 24) aufweist, die bei jedem Atemzyklus Licht- oder Schallimpulse erzeugen können.

11. Gerät nach einem der vorhergehenden Ansprüche, dadurch gekennzeichnet, dass es ausserdem eine Vorrichtung (19) zum Wecken oder zur Wiederbelebung des Kindes aufweist, die von einem vom autonomen Erfassungs- und Anzeigesystem abgegebenen Signal ausgelöst wird, wenn eine Atempause eine vorbestimmte Dauer überschreitet.

12. Gerät nach Anspruch 11, dadurch gekennzeichnet, dass besagte Weck- und Wiederbelebungsvorrichtung mindestens eine Elektrode (19) aufweist, die auf der Aussenseite (11) der Auflagewandung des Gehäuses (1) augebracht ist und durch die vom besagten Signal gesteuerte elektrische Entladungen erzeugt werden können.

13. Gerät nach einem der vorhergehenden Ansprüche, dadurch gekennzeichnet, dass es einen, im Gehäuse angebrachten, Hochfrequenzsender aufweist, der das von besagten Erfassungsorganen erzeugte Signal und/oder besagtes Alarmsignal an mindestens einen Empfänger weitergibt, welcher der Person zugeordnet ist, die das Kind überwacht.

14. Gerät nach einem der vorhergehenden Ansprüche, dadurch gekennzeichnet, dass es ausserdem Mittel enthält zur:
- Bestimmung des Zeitraums zwischen dem Atemstillstand und der Abgabe des Alarmsignals; und/oder
- Bestimmung des Zeitraums zwischen dem Atemstillstand und der Auslösung der Vorrichtung zum Wecken des Kindes und/oder
- Hemmung des bei jedem Atemsyklus des Kindes abgegebenen akustischen Signals; und/oder
- automatischen Freisetzung (Abschaltung, Wiedereinschaltung) des akustischen Alarms und/oder der Vorrichtung zum Wecken des Kindes, infolge eines neuen Atemzuges; und/oder
- Hemmung der Vorrichtung zum Wecken des Kindes; und/oder
- Zählung und Anzeige auf Wunsch der Zahl der Atempausen, die einen vorbestimmten Zeitraum überschreiten, mit der Möglichkeit, den Zähler auf Null rückzustellen; und/oder
- Kontrolle des Zustandes der benutzten Batterie, mit akustischem Alarm, falls diese verbraucht ist; und/oder
- Kontrolle des guten Funktionierens der Elektronik, durch einen unabhängigen elektronischen Kreis, mit akustischem Alarm im Falle einer Funktionsstörung.

**Claims**

1. Apparatus for the continuous detection of a child's breathing rhythm, in particular with a view of preventing the sudden death of the infant by the cessation of breathing during sleep, this apparatus comprising a device for detecting and signalling the breathing rhythm of the child and equipped with means enabling an abnormal breathing pause to be detected and an alarm signal to be generated when this pause exceeds a predetermined period, characterized in that:

- the detection and signalling device is self-contained and housed inside a small box (1) comprising an application wall whose outer face is designed to be kept applied against the skin of the child in a suitably selected place, the said wall comprising at least one detection zone (15) moving under the effect of displacement of the skin generated by the child's breathing process;

- this detection and signalling device includes a transducer (12, 13, 18) sensitive to the movements of the detection zone;

- the box (1) is equipped with fasteners (8, 9, 28, 29) to be connected to the body of the child and which are able to keep the said outer face (2) of the application wall and, in particular, that of the detection zone (15), against the skin of the child.

2. Apparatus according to claim 1,
characterized in that the above-mentioned transducer (12) includes an electret transmitter on which are retransmitted, by transmission means, the displacements and/or pressure variations exerted whilst the child's skin is in contact with the application surface of the box (1).

3. Apparatus according to claim 2,
characterized in that the said means of transmission are mechanical or hydraulic.

4. Apparatus according to one of claims 2 and 3,
characterized in that the said transducer and the said means of transmission are housed inside a recess (11) formed inside the application wall and reclosed at the outer face (2) by a flexible diaphragm (5).

5. Apparatus according to claim 4,
characterized in that the said means of transmission include a first mobile thruster (13) coming to rest against the said transducer (12) and a second thruster (14) kept applied against the said diaphragm (15) and in that a spring (18) is disposed between the two thrusters (13, 14).

6. Apparatus according to one of the preceding claims,
characterized in that the said means of fastening of the box (1) consist of an adhesive (9) provided on the periphery of the application face (11) of the box (1) and designed to act directly on the child's skin.

7. Apparatus according to claim 6,
characterized in that the said adhesive (9) is applied to a removable part (8) coming to rest on the application wall (11) of the box (1).

8. Apparatus according to claim 7,
characterized in that the said part consists of a ring (8) screwed into a thread fitted on a shoulder (7) of the outer face (11) of the application wall of the box (1).

9. Apparatus according to one of the preceding claims,
characterized in that the box (1) includes means (28) for fastening an abdominal belt (29).

10. Apparatus according to one of the preceding claims,
characterized in that the above-mentioned signalling and dedetection device includes signalling means (22, 23, 24) provided inside the box enabling light and/or sound pulses to be produced with each breathing cycle.

11. Apparatus according to one of the preceding claims,
characterized in that it includes also a waking or resuscitation device (19) for rousing the child and controlled by a signal transmitted by the self-contained detection and signalling device when a breathing pause exceeds a predetermined period.

12. Apparatus according to claim 11,
characterized in that the said resuscitation or waking device comprises at least one electrode (19) placed on the outer face (11) of the application wall of the box (1) and through which can be produced electrical discharges controlled by the said signal.

13. Apparatus according to one of the preceding claims,
characterized in that it includes a high frequency emitter placed inside the box which is designed to transmit the signal produced by the said detection devices and/or the above-mentioned alarm signal to at least one receiver carried by the person monitoring the child.

14. Apparatus according to one of the preceding claims,
characterized in that it also includes means for:

- the preselection of the time interval chosen between the cessation of breathing and the emission of the alarm signal; and/or

- the preselection of the time interval chosen between cessation of breathing and the triggering of the device for waking the child; and/or

- the inhibition of the sound signal emitted with each breathing cycle of the child; and/or

- the automatic discharge (stoppage/setting) of the sound alarm and/or device for waking the child following renewed breathing; and/or

- the inhibition of the device for waking the child; and/or·

- the counting and display on demand of the number of respiratory pauses greater than a preselected time interval with the possibility of resetting the counter; and/or

- the inspection of the state of the battery used with sound alarm in the event of wear and tear of the latter; and/or

- ensuring proper electronic functioning by an independent electronic circuit, with sound alarm in the event of malfunction.

EP 0 178 197 B1

FIG.1

FIG.2

FIG.3

VENTRE

FIG.4

VENTRE

1